# EUROPEAN PATENT SPECIFICATION

(11) **EP 0 928 190 B1**
(45) Date of publication and mention of the grant of the patent: **17.11.2004**
(21) Application number: 97919142.6
(22) Date of filing: 11.09.1997
(51) Int. Cl.: A61K 9/16, A61K 9/14, A61K 48/00

(54) **NUCLEIC ACID PARTICLE DELIVERY**
VERABREICHUNG VON NUKLEINSÄUREPARTIKELN
ADMINISTRATION DE PARTICULES D'ACIDE NUCLEIQUE

(30) Priority: 11.09.1996 GB 9619002
(43) Date of publication of application: 14.07.1999
(73) Proprietor: PowderJect Research Limited, Oxford OX4 4GA (GB)
(72) Inventor: BURKOTH, Terry, Lee, Palo Alto, CA 94303 (US); MUDDLE, Andrew, Gordon, Cambs. CB6 1AN (GB); PORTER, Linda, Maree, The Gap, QLD 4061 (AU)
(74) Representative: Woods, Geoffrey Corlett
(86) International application number: PCT/GB1997/002478
(87) International publication number: WO 1998/010750

(56) References cited:
- WO-A-94/23738
- WO-A-97/48485

## Description

### Technical Field

The present invention relates generally to DNA delivery methods. More particularly, the invention pertains to *in vivo* and *ex vivo* delivery of powdered nucleic acid molecules into mammalian tissue using needleless injection techniques.

### Background of the Invention

Gene therapy and DNA immunization are promising approaches for the treatment and prevention of both acquired and inherited diseases. These techniques provide for the transfer of a desired gene into a subject with the subsequent in vivo expression thereof. Gene transfer can be accomplished by transfecting the subject's cells or tissues *ex vivo* and reintroducing the transformed material into the host. Alternatively, genes can be administered directly to the recipient.

A number of methods have been developed for gene delivery in these contexts. For example, viral-based systems using, e.g., retrovirus, adenovirus, and adeno-associated viral vectors, have been developed for gene delivery. However, these systems pose the risk of delivery of replication-competent viruses. Hence, nonviral methods for direct transfer of genes into recipient cells and tissues are desirable.

Nonviral methods of gene transfer often rely on mechanisms employed by mammalian cells for the uptake and intracellular transport of macromolecules. For example, receptor-mediated methods of gene transfer have been developed. The technique utilizes complexes between plasmid DNA and polypeptide ligands that can be recognized by cell surface receptors. However, data suggests that this method may permit only transient expression of genes and thus has only limited application.

Additionally, microinjection techniques have been developed for the direct injection of genetic material into cells. The technique, however, is laborious and requires single cell manipulations. Thus, the method is inappropriate for use on a large scale.

Direct injection of DNA-containing solutions into the interstitial space for subsequent uptake by cells has also been described. For example, International Publication No. WO 90/11092, published 4 October 1990, describes the delivery of isolated polynucleotides to the interior of cells wherein the isolated polynucleotides are delivered into the interstitial space of the tissue and then taken up by individual cells to provide a therapeutic effect. Such methods entail the injection of the DNA-containing solutions into tissue using conventional needles or cannulas, and are therefore not well suited for long term therapies or for field or home applications.

Biolistic particle delivery systems (particle bombardment systems) have also been developed for gene delivery into plant cells. Such techniques use a "gene gun" to introduce DNA-coated microparticles, such as DNA-coated metals, into cells at high velocities. The coated metals are generally propelled into cells using an explosive burst of an inert gas such as helium. See, e.g., U.S. Patent No. 5,100,792 to Sanford et al. The technique allows for the direct, intracellular delivery of small amounts of DNA.

Tungsten or gold particle microprojectiles are generally needed to achieve adequate gene transfer frequency by such direct injection techniques. In particular, these materials have been selected based on their availability in defined particle sizes around 1 µm in diameter, as well as having a sufficiently high density to achieve the momentum required for cell wall penetration. Additionally, the metals used are chemically inert to reduce the likelihood of explosive oxidation of fine microprojectile powders, as well as non-reactive with DNA and other components of the precipitating mixes, and display low toxicity to target cells. See e.g., *Particle Bombardment Technology for Gene Transfer,* (1994) Yang. N. ed., Oxford University Press, New York, NY pages 10-11.

However, there is evidence that tungsten toxicity can reduce the recovery of stable transformants. Additionally, such biolistic techniques are not appropriate for use with large DNA molecules since precipitation of such molecules onto metal carriers can lead to unstable configurations which will not withstand the shear forces of gene gun delivery. Furthermore, metal carriers are retained by tissues and/or cells and may cause discoloration, as well as a number of other undesirable biological effects particularly in the case of direct delivery to internal tissues, or direct treatment of cells using *ex vivo* techniques. Thus, the use of a gene gun to deliver DNA-coated metal particles is likely to be problematic for repeated therapy, especially in mammalian subjects.

WO 94/23738 is concerned with microparticles suitable for controlled release of a nucleic acid to a target cell. The microparticles are composed of a number of components including a nucleic acid, a material which promotes the uptake or transport to the nucleus of the nucleic acid or expression of the nucleic acid and a specific biocompatible biodegradable polymeric matrix. The material that promotes uptake, transport or expression may be a glycoprotein or lipoprotein. The polymeric matrix encapsulates the nucleic acid.

Accordingly, there remains a need to provide a highly efficient method for introducing therapeutically relevant DNA or other nucleic acid molecules into mammalian tissue cells wherein the method avoids the problems commonly encountered with prior gene delivery techniques.

### Disclosure of the Invention

According to the present invention, there is provided use of a nucleic acid molecule and a carbohydrate as sole carrier for the manufacture of a medicament in the form of particles for use in therapy by needleless administration to skin or mucosal tissue, wherein
- the nucleic acid molecule comprises a nucleotide sequence encoding an immunogen,
- control sequences capable of effecting the expression of said nucleotide sequence are operably linked thereto, and
- said particles have a size ranging from 10 to 150 µm and a density ranging from 0.8 to 3.0 g/cm³.

The invention also provides a needleless syringe comprising, as the active component to be delivered, particles as defined above.

The present invention is based on the surprising discovery that solid particles of nucleic acid molecules having a nominal average diameter of at least about 10 µm and which are therefore larger than the average mammalian cell, can be delivered into the cells of mammalian tissue for highly efficient gene transfer. The result is unexpected because it was heretofore believed that only small DNA-coated metallic particles, having a much smaller size than typical mammalian cells, could adequately be used as microprojecticles in biolistic gene delivery techniques. See e.g. *Particle Bombardment Technology for Gene Transfer,* (1994) Yang, N. ed., Oxford University Press, New York, NY pages 10-11.

In the practice of the invention, powdered nucleic acid molecules are delivered using needleless injection techniques. In particular, a novel delivery system that uses a needleless syringe to fire solid particles of therapeutic agents in controlled doses into and through intact skin has recently been described in commonly assigned International Publication No. WO 94/24263, published 27 October 1994. The publication describes a needleless syringe that delivers pharmaceutical particles entrained in a supersonic gas flow. The needleless syringe can be used for transdermal delivery of powdered drug compounds and compositions, for delivery of genetic material into living cells, (e.g. gene therapy) and for the delivery of biopharmaceuticals to skin, muscle, blood or lymph. The needleless syringe can also be used in conjunction with surgery to deliver drugs and biologics to organ surfaces, solid tumors and/or to surgical cavities (e.g., tumor beds or cavities after tumor resection).

Accordingly, in one embodiment, solid particles comprised of nucleic acid molecules are delivered to mammalian tissue for the genetic transformation of cells in the tissue with the delivered nucleic acids. In a substantial departure from conventional particle bombardment techniques, the nucleic acid particles transferred using the method of the present invention are not delivered using dense metal carriers. Furthermore, the molecules have a particle size that is equal to or larger than the average mammalian cell size.

More particularly, densified particles comprised of selected nucleic acid molecules and, a carbohydrate carrier are prepared for delivery to mammalian tissue via a needleless syringe which is capable of expelling the particles at supersonic delivery velocities of between Mach 1 and Mach 8. The particles have an average size that is at least about 10 µm, wherein an optimal particle size is usually at least about 10 to 15 µm (equal to or larger than the size of a typical mammalian cell). However nucleic acid particles having average particle sizes up to 50 µm can also be delivered using the present method. The depth that the delivered particles will penetrate the targeted tissue depends upon particle size (e.g., the nominal particle diameter assuming a roughly spherical particle geometry), particle density, the initial velocity at which the particle impacts the tissue surface, and the density and kinematic viscosity of the tissue. In this regard, optimal individual particle densities (e.g., in contrast to bulk powder density) for use in needleless injection range between about 0.8 and 3.0 g/cm³, and injection velocities generally range between about 200 and 3,000 m/sec.

The invention can be practised *in vivo* to provide targeted delivery of the nucleic acid particles, such as delivery to the epidermis (for gene therapy applications) or to the *stratum basal* layer of skin (for nucleic acid immunization applications). In these aspects, particle characteristics and/or device operating parameters are selected to provide tissue-specific delivery. One particular approach entails the selection of particle size, particle density and initial velocity to provide a momentum density (e.g., particle momentum divided by particle frontal area) of between about 2 and 10 kg/sec/m, and more preferably between about 4 and 7 kg/sec/m. Such control over momentum density allows for precisely controlled, tissue-selective delivery of the nucleic acid particles.

In other aspects , the needleless syringe is used to transfect cells or tissues *ex vivo* with the particulate nucleic acid molecules, wherein the transformed cells are subsequently reintroduced into the host.

These and other embodiments of the subject invention will readily occur to those of skill in the art in light of the disclosure herein.

### Brief Description of the Figures

Figure 1 is a pictorial representation of an ex vivo delivery apparatus having a needleless syringe arranged over a tissue culture plate containing cells to be transformed with the particulate nucleic acid preparations described herein.
Figure 2 is a histogram depicting transformation efficiencies obtained using the apparatus of Figure 1 to deliver DNA particles at 30 bar pressure over a 60 mm target distance as described in the Example.
Figure 3 is a graph depicting the transformation efficiencies obtained using the apparatus of Figure 1 to deliver DNA particles at 30 bar pressure over a range of target distances, also as described in the Example.

### Detailed Description of the Preferred Embodiments

The practice of the present invention will employ, unless otherwise indicated, conventional methods of molecular biology and recombinant DNA techniques within the skill of the art. Such techniques are explained fully in the literature. See, e.g., Sambrook, et al., *Molecular Cloning: A Laboratory Manual* (2nd Edition, 1989); Maniatis et al., *Molecular Cloning: A Laboratory Manual* (1982); DNA Cloning: A Practical Approach, vol. I & II (D. Glover, ed.); Perbal, A Practical Guide to Molecular Cloning.

It must be noted that, as used in this specification and the appended claims, the singular forms "a", "an" and "the" include plural referents unless the content clearly dictates otherwise. Thus, for example, reference to "a nucleic acid molecule" includes a mixture of two or more nucleic acid molecules, reference to "an excipient" includes mixtures of two or more excipients, and the like.

### A. Definitions

Unless defined otherwise, all technical and scientific terms used herein have the same meaning as commonly understood by one of ordinary skill in the art to which the invention pertains. The following terms are intended to be defined as indicated below.

"Gene delivery" refers to methods or systems for reliably inserting foreign DNA into host cells. such methods can result in expression of non-integrated transferred DNA, extrachromosomal replication and expression of transferred replicons (e.g., episomes), or integration of transferred genetic material into the genomic DNA of host cells.

The nucleotide sequences are generally present in a suitable nucleic acid molecule and delivered in the form of vectors. By "vector" is meant any genetic element, such as a plasmid, phage, transposon, cosmid, chromosome, virus, virion, etc., which is capable of replication when associated with the proper control elements and which can transfer gene sequences between cells.

A "nucleotide sequence" or a "nucleic acid molecule" refers to DNA and RNA sequences. The term captures molecules that include any of the known base analogues of DNA and RNA such as, but not limited to 4-acetylcytosine, 8-hydroxy-N6-methyladenosine. aziridinylcytosine, pseudoisooytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

A "coding sequence" or a sequence which "encodes" a particular polypeptide, is a nucleic acid sequence which is transcribed (in the case of DNA) and translated (in the case of mRNA) into a polypeptide *in vitro* or *in vivo* when placed under the control of appropriate regulatory sequences. The boundaries of the coding sequence are conventionally determined by a start codon at the 5' (amino) terminus and a translation stop codon at the 3' (carboxy) terminus. A coding sequence can include, but is not limited to, cDNA from procaryotic or eukaryotic mRNA, genomic DNA sequences from procaryotic or eukaryotic DNA, and even synthetic DNA sequences. A transcription termination sequence will usually be located 3' to the coding sequence.

The term DNA "control sequences" refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected gene is capable of being replicated, transcribed and translated in an appropriate recipient cell.

"Operably linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

By "isolated" when referring to a nucleotide sequence, or a nucleic acid molecule containing the nucleotide sequence, is meant that the indicated molecule is present in the substantial absence of other biological macromolecules of the same type. Thus, an "isolated nucleic acid molecule which encodes a particular polypeptide" refers to a nucleic acid molecule which is . substantially free of other nucleic acid molecules that do not encode the subject polypeptide; however, the molecule may include some additional bases or moieties which do not deleteriously affect the basic characteristics of the composition.

The term "transfection" is used to refer to the uptake of foreign DNA by a host cell, and a host cell has been "transformed" as a result of having been transfected. The foreign DNA may or may not be integrated (covalently linked) to chromosomal DNA making up the genome of the cell. By "host cell," or "host mammalian cell" is meant a cell which has been transfected, or is capable of being transfected, by a nucleic acid molecule containing a nucleotide sequence of interest. The term includes the progeny of the parent cell, whether or not the progeny is identical in morphology or in genetic make-up to the original parent, so long as the nucleotide sequence of interest is present within the cell.

The term "transdermal" delivery captures both transdermal (or "percutaneous") and transmucosal administration, i.e., delivery by passage of a drug or pharmaceutical agent through the skin or mucosal tissue See, e.g., *Transdermal Drug Delivery: Developmental Issues and Research Initiatives,* Hadgraft and Guy (eds.), Marcel Dekker, Inc., (1989); *Controlled Drug Delivery: Fundamentals and Applications*, Robinson and Lee (eds.), Marcel Dekker Inc., (1987); and *Transdermal Delivery of Drugs*, Vols. 1-3, Kydonieus and Berner (eds.), CRC Press, (1987). Aspects of the invention which are described herein in the context of "transdermal" delivery, unless otherwise specified, are meant to apply to both transdermal and transmucosal delivery. That is, the compositions, systems, and methods of the invention, unless explicitly stated otherwise, should be presumed to be equally applicable to transdermal and transmucosal modes of delivery.

The above nucleic acid molecules, alone or in combination with drugs or other therapeutic agents, are typically prepared as particular compositions which contain a carbohydrate as the sole (excipient). "Carriers" are normally substantially inert materials which are nontoxic and do not interact with other components of the composition in a deleterious manner. These materials can be used to increase the amount of solids in particular pharmaceutical compositions, such as those prepared using spray-drying or lyophilization techniques, as described further below. The sole carrier material in the present instance is a carbohydrate. Examples include pharmaceutical grades of dextrose, sucrose, lactose, trehalose, mannitol, sorbitol, inositol, dextran, starch and cellulose. Also present may be additive agents that serve as stabilizers include commonly available cryoprotectants and antioxidants.

### B. Modes of Carrying Out the Invention

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

As explained above, the present invention allows for the highly efficient delivery of solid particles of nucleic acid molecules having a nominal average diameter of at least about 10 µm, to mammalian tissues and cells. The method utilizes biolistic gene transfer techniques yet allows for the delivery of nucleic acid molecules without the need for metal carriers.

A wide variety of nucleic acid molecules can be delivered using the methods of the invention. Generally, the molecules contain coding regions with suitable control sequences or other therapeutically relevant nucleotide sequences. The nucleic acid molecules are prepared in the form of vectors which include the necessary elements to direct transcription and translation in a host cell. If expression is desired using the host's enzymes (such as by the use of endogenous RNA polymerase), the gene or genes will be present in the vectors operatively linked to control sequences recognized by the particular host, or even particular cells within the host. Thus, eucaryotic and phage control elements will be present for expression in mammalian hosts. Such sequences are known in the art and are discussed more fully below.

Suitable nucleotide sequences for use in the delivery methods of the present invention are any therapeutically relevant nucleotide sequence that encode an immunogen. The invention is thus used to deliver a nucleotide sequence capable of providing immunity, for example an immunogenic sequence that serves to elicit a humoral and/or cellular response in a subject.

For nucleic acid immunizations, antigen-encoding expression vectors can be delivered to a subject for the purpose of eliciting humoral and/or cellular immune responses to antigens encoded by the vector. In particular, humoral, cytotoxic cellular and protective immune responses elicited by direct intramuscular injection of antigen-encoding DNAs have been described. Tang et al. (1992) *Nature* 358:152; Davis et al. (1993) Hum. Molec. Genet. 2:1847; Ulmer et al. (1993) *Science* 258:1745; Wang et al. (1993) *Proc. Natl*. *Acad. Sci. USA* 90:4156; Eisenbraun et al. (1993) *DNA Cell Biol.* 12:791; Fynan et al. (1993) *Proc*. *Natl. Acad. Sci. USA* 90:12476; Fuller et al. (1994) *AIDS Res. Human Retrovir.* 10:1433; and Raz et al. (1994) *Proc. Natl. Acad. Sci. USA* 91:9519.

Modes of carrying out the invention are described more fully below.

### Isolation of Genes and Construction of Vectors:

Nucleotide sequences selected for use in the present invention can be derived from known sources, for example, by isolating the same from cells containing a desired gene or nucleotide sequence using standard techniques. Similarly, the nucleotide sequences can be generated synthetically using standard modes of polynucleotide synthesis that are well known in the art. See, e.g., Edge et al. (1981) *Nature* 292:756; Nambair et al. (1984) *Science* 223:1299; Jay et al. (1984) *J. Biol. Chem.* 259:6311. Generally, synthetic oligonucleotides can be prepared by either the phosphotriester method as described by Edge et al. (*supra*) and Duckworth et al. (1981) *Nucleic Acids Res.* 9:1691, or the phosphoramidite method as described by Beaucage et al. (1981) *Tet. Letts.* 22:1859, and Matteucci et al. (1981) *J. Am. Chem. Soc.* 103:3185. Synthetic oligonucleotides can also be prepared using commercially available automated oligonucleotide synthesizers. The nucleotide sequences can thus be designed with appropriate codons for a particular amino acid sequence. In general, one will select preferred codons for expression in the intended host. The complete sequence is assembled from overlapping oligonucleotides prepared by standard methods and assembled into a complete coding sequence. *See*, *e*.*g*., Edge et al. (*supra*); Nambair et al. (*supra*) and Jay et al. (*supra*).

A particularly convenient method for obtaining nucleic acid sequences for use herein is by recombinant means. Thus, a desired nucleotide sequence can be excised from a plasmid carrying the same using standard restriction enzymes and procedures. Site specific DNA cleavage is performed by treating with the suitable restriction enzyme (or enzymes) under conditions which are generally understood in the art, and the particulars of which are specified by manufacturers of commercially available restriction enzymes. If desired, size separation of the cleaved fragments may be performed by polyacrylamide gel or agarose gel electrophoresis using standard techniques.

Restriction cleaved fragments may be blunt ended by treating with the large fragment of E. coli DNA polymerase I (Klenow) in the presence of the four deoxynucleotide triphosphates (dNTPs) using standard techniques. The Klenow fragment fills in at 5' single-stranded overhangs but digests protruding 3' single strands, even though the four dNTPs are present. If desired, selective repair can be performed by supplying only one, or several, selected dNTPs within the limitations dictated by the nature of the overhang. After Klenow treatment, the mixture can be extracted with e.g. phenol/chloroform, and ethanol precipitated. Treatment under appropriate conditions with S1 nuclease or BAL-31 results in hydrolysis of any single-stranded portion.

Once coding sequences for desired proteins have been prepared or isolated, such sequences can be cloned into any suitable vector or replicon. Numerous cloning vectors are known to those of skill in the art, and the selection of an appropriate cloning vector is a matter of choice. Ligations to other sequences are performed using standard procedures, known in the art.

Selected nucleotide sequences can be placed under the control of regulatory sequences such as a promoter, ribosome binding site and, optionally, an operator (collectively referred to herein as "control" elements), so that the sequence encoding the desired protein is transcribed into RNA in the host tissue transformed by a vector containing this expression construct. The coding sequence may or may not contain a signal peptide or leader sequence.

The choice of control elements will depend on the host being transformed and the type of preparation used. Thus, if the host's endogenous transcription and translation machinery will be used to express the proteins, control elements compatible with the particular host will be utilized. In this regard, several promoters for use in mammalian systems are known in the art and include, but are not limited to, promoters derived from SV40, CMV, HSV, RSV, MMTV, T7, T3, among others. Similarly, promoters useful with procaryotic enzymes are known and include the tac, spa, trp, trp-lac λ-p_{L}, T7, phoA promoters, as well as others.

In addition to control sequences, it may be desirable to add regulatory sequences which allow for regulation of the expression of protein sequences encoded by the delivered nucleotide sequences. Regulatory sequences are known to those of skill in the art, and examples include those which cause the expression of a coding sequence to be turned on or off in response to a chemical or physical stimulus, including the presence of a regulatory compound. Other types of regulatory elements may also be present in the vector, for example, enhancer sequences.

An expression vector is constructed so that the particular coding sequence is located in the vector with the appropriate control and, optionally, regulatory sequences such that the positioning and orientation of the coding sequence with respect to the control sequences allows the coding sequence to be transcribed under the "control" of the control sequences (i.e., RNA polymerase which binds to the DNA molecule at the control sequences transcribes the coding sequence). Modification of the sequences encoding the particular protein of interest may be desirable to achieve this end. For example, in some cases it may be necessary to modify the sequence so that it is attached to the control sequences with the appropriate orientation; i.e., to maintain the reading frame. The control sequences and other regulatory sequences may be ligated to the coding sequence prior to insertion into a vector. Alternatively, the coding sequence can be cloned directly into an expression vector which already contains the control sequences and an appropriate restriction site.

### Preparation of Particulate Nucleic Acid Molecules:

Once obtained and/or constructed, the nucleic acid molecules are prepared for delivery in particulate form. One common method of preparing particulate biopharmaceuticals, such as nucleic acids, is lyophilization (freeze-drying). Lyophilization relates to a technique for removing moisture from a material and involves rapid freezing at a very low temperature, followed by rapid dehydration by sublimation in a high vacuum. This technique typically yields low-density porous particles having an open matrix structure. Such particles are chemically stable, but are rapidly reconstituted (disintegrated and/or brought into solution) when introduced into an aqueous environment.

Another method of providing particulate nucleic acid preparations that can be used with these and other delicate or heat-sensitive biomolecules is spray-drying. Spray-drying relates to the atomization of a solution of one or more solids using a nozzle, spinning disk or other device, followed by evaporation of the solvent from the droplets. More particularly, spray-drying involves combining a highly dispersed liquid preparation (e.g., a solution, slurry, emulsion or the like) with a suitable volume of hot air to produce evaporation and drying of the liquid droplets. Spray-dried pharmaceuticals are generally characterized as homogenous spherical particles that are frequently hollow. Such particles have low density and exhibit a rapid rate of solution.

The low-density particulate solids produced by lyophilization and spray-drying techniques are ideal for redissolution for parenteral administration in solution via syringe or catheters. However, such particles are not useful for delivery from a needleless syringe in a solid form. Accordingly, for purposes of the present method, the preparations are densified to provide particles including nucleic acid molecules that are much better suited for delivery using a needleless syringe (e.g., substantially solid particles having a size of about 50 µm and a density of at least about 0.9 to 1.5 g/cc³). In particular, the open lattice or hollow shell particles provided by spray-drying or lyophilization can be condensed without heating or shear to provide dense materials that can be milled or otherwise size-reduced to yield pharmaceutical particles having both size and density characteristics suitable for delivery by needleless injection.

The nucleic acids for delivery in accordance with the invention are initially prepared in a formulation suitable for spray-drying or lyophilization. Such formulations generally require only a solution in which the nucleic acids will be stable for freezing and lyophilization and a carbohydrate carrier (excipient) for the drying procedure which is acceptable for parenteral delivery. In this regard, suitable carbohydrate excipients may be added to the formulations to provide sufficient mass for an individual dose, enabling measurement of doses by practical processes, e.g., by weight or volume. Typical dosages can be about 0.5 to about 5 mg, preferably about 1 to about 2 mg. Suitable excipients include, but are not limited to, trehalose, glucose, dextrose, sucrose and mannitol. Amino acids such as glycine and its hydrochloride salt can be used as buffers as well as phosphate, lactate or citrate buffers, among others. Additionally, any known composition for DNA stabilization will find use in the present formulations. The compositions may optionally include additive agents such as cryoprotectants, antioxidants, or the like. Adjusting compositions to enhance physical and chemical stability of the various particulate nucleic acid formulations provided herein is within the ordinary skill in the art.

One particular approach to stabilization during reprocessing of the nucleic acid formulations entails the use of additives which are combined with the solution prior to freezing for lyophilization to cause the nucleic acids to coil or ball and thus provide the genetic material as a discontinuous phase in the otherwise microscopically homogeneous particles. In such formulations, the bulking agent would be the continuous phase in the dried solid so that any grinding prior to compression, compression densification and regrinding (as described in detail below), and any particle attrition during sizing via sieve or air classification, acceleration and injection, would be less likely to disrupt the long chain nucleic acids. Homogeneity of the particles with respect to nucleic acid content is critical because of the potential for segregation by size during storage or injection.

Condensing the spray-dried or lyophilized powders is conducted by compaction in a suitable press (e.g., a hydraulic press, tableting press or rotary press), wherein the powders are compressed at about 1,000 to 24,000 pounds/square inch (e.g., 0.5 to 12 tons/square inch or 7 to 170 MPa) for a suitable time. Compaction can be carried out under vacuum if desired. The resulting compacted material is then coarsely reground until visually broken up. The particle size is then reduced to about a 20 to 50 µm average size with an individual particle density of around 0.9 to 1.5 g/cm³. Particle size reduction can be conducted using methods well known in the art including, but not limited to, roller milling, ball milling, hammer or impact milling, attrition milling, sieving, sonicating, or combinations thereof. The compression parameters and particle sizing will, of course, vary depending upon the starting material used, the desired target particle size and density, and like considerations. Particle density can be readily ascertained using known quantification techniques such as helium pycnometry and the like.

Thus, the method can be used to obtain nucleic acid particles having a size ranging from 10 to 150 µm, and most preferably 20 to 60 µm; and a particle density ranging from 0.8 to 3.0 g/cm³, and preferably 0.9 to 1.5 g/cm³.

### Administration of the Nucleic Acid Molecules:

Following formation, the particulate nucleic acid preparations are delivered to mammalian tissue using a needleless syringe. One needleless syringe for use herein generally is described in commonly assigned International Publication No. WO 94/24263, published 27 October 1994. The syringe comprises an elongate tubular nozzle having a rupturable membrane (or membranes) initially closing the passage through the nozzle and arranged substantially adjacent to the upstream end of the nozzle. The nucleic acid particles to be delivered are disposed adjacent to the rupturable membrane and are delivered using an energizing means which applies a gaseous pressure to the upstream side of the membrane sufficient to burst the membrane and produce a supersonic gas flow (containing the pharmaceutical particles) through the nozzle for delivery from the downstream end thereof. The particles can thus be delivered from the needleless syringe at delivery velocities of between Mach 1 and Mach 8 which are readily obtainable upon the bursting of the rupturable membrane.

Another needleless syringe configuration generally includes the same elements as described above, except that instead of having the pharmaceutical particles entrained within a supersonic gas flow, the downstream end of the nozzle is provided with a bistable diaphragm which is moveable between a resting "inverted" position (in which the diaphragm presents a concavity on the downstream face to contain the nucleic acid particles) and an active "everted" position (in which the diaphragm is outwardly convex on the downstream face as a result of a supersonic shockwave having been applied to the upstream face of the diaphragm). In this manner, the particles contained within the concavity of the diaphragm are expelled at a supersonic initial velocity from the device for transdermal delivery thereof to a targeted skin or mucosal surface.

Transdermal delivery using the above-described needleless syringe configurations is carried out with particles having an approximate size that generally ranges between 10 and 150 µm. The optimal particle size is usually at least about 10 to 15 µm (the size of a typical cell). Nucleic acid particles larger than about 250 µm can also be delivered from the device, with the upper limitation being the point at which the size of the particles would cause untoward damage to the skin. The actual distance which the delivered particles will penetrate depends upon particle size (e.g., the nominal particle diameter assuming a roughly spherical particle geometry), particle density, the initial velocity at which the particle impacts the skin surface, and the density and kinematic viscosity of the skin. In this regard, particle densities for use in needleless injection range between 0.8 and 3.0 g/cm³, preferably between 0.9 and 1.5 g/cm³, and injection velocities generally range between 200 and 3,000 m/sec.

A particularly unique feature of the needleless syringe is the ability to closely control the depth of penetration of delivered particles, thereby allowing for targeted administration of the nucleic acids to various sites. For example, particle characteristics and/or device operating parameters can be selected to provide penetration depths of less than about 1 mm for intradermal delivery, or approximately 1-2 mm for subcutaneous delivery. One approach entails the selection of particle size, particle density and initial velocity to provide a momentum density (e.g., particle momentum divided by particle frontal area) of between about 2 and 10 kg/sec/m, and more preferably between about 4 and 7 kg/sec/m. Such control over momentum density allows for precisely controlled, tissue-selective delivery of the particulate nucleic acids.

Compositions containing a therapeutically effective amount of the powdered nucleic acid molecules described herein can be delivered to any suitable mammalian tissue via the above-described needleless syringes. For example, the compositions can be delivered to muscle, skin, brain, lung, liver, spleen, bone marrow, thymus, heart, lymph, blood, bone cartilage, pancreas, kidney, gall bladder, stomach, intestine, testis, ovary, uterus, rectum, nervous system, eye, gland and connective tissues. The nucleic acid molecules are preferably delivered to, and expressed in, terminally differentiated cells; however, the molecules can also be delivered to non-differentiated, or partially differentiated cells such as stem cells of blood and skin fibroblasts.

The powdered compositions are administered to the subject to be treated in a manner compatible with the dosage formulation, and in an amount that will be prophylactically and/or therapeutically effective. The amount of the composition to be delivered, generally in the range of from 0.5 µg/kg to 100 µg/kg of nucleic acid molecule per dose, depends on the subject to be treated. The exact amount necessary will vary depending on the age and general condition of the individual to be treated, the severity of the condition being treated and the particular nucleotide sequence or sequences selected, the site of administration, as well as other factors. An appropriate effective amount can be readily determined by one of skill in the art.

Thus, a "therapeutically effective amount" of the present powdered nucleic acid molecule compositions will be sufficient to bring about prevention of disease or condition symptoms, and will fall in a relatively broad range that can be determined through routine trials.

### C. Experimental

Below are Reference Examples that are offered for illustrative purposes only, and are not intended to limit the scope of the present invention in any way.

Efforts have been made to ensure accuracy with respect to numbers used (*e*.*g*., amounts, temperatures, etc.), but some experimental error and deviation should, of course, be allowed for.

### Reference Example 1

The following experiment was conducted to investigate the possibility of using freeze-dried DNA as an alternative to DNA-coated metal particles in the biolistic transfer of genetic material. In particular, powdered DNA plasmids as well as DNA-coated tungsten particles as controls were delivered ex vivo to male human fibroblast HT1080 cells using a needleless syringe apparatus as follows.

Clone 123 is a small plasmid of -11 kb which contains the β-galactosidase marker gene so that transient transformation can be measured with the chromogenic indicator X-Gal. Plasmids were bulked with a carbohydrate excipient, trehalose. Trehalose was selected as the excipient because of its stabilizing properties (Colaco et al. (1992) *Bio*/*Technology* 10:1009). The trehalose was dissolved in distilled water and filter-sterilized prior to adding the DNA to the solution. Three different solutions of DNA sugar were made up with the proportions shown below in Table 1.

**Table 1**

| Preparation | 1 | 2 | 3 |
|---|---|---|---|
| Clone 123 (2.7µg/µL) | 800 µg (296.3 µL) | 160 µg (59.3 µL) | 80 µg (29.6 µL) |
| Trehalose (100 mg/15 ml H₂O) | 10 mg (1.5 ml) | 10 mg (1.5 ml) | 10 mg (1.5 ml) |
| Payload (for 8 µg DNA) | 0.1 mg | 0.5 mg | 1-0 mg |

The plasmid/sugar solutions were then freeze-dried (using solid CO₂ and isopropanol to freeze the solution prior to vacuum drying), and the freeze-dried DNA-trehalose solid milled to form microparticles using an agate mortar and pestle.

As a control, DNA-coated tungsten particles were made by coating tungsten microprojectiles (19.35 x 10³ kb.m⁻³) of 1.014 µm median diameter (M-17, GTE/Sylvania, Towanda, PA, USA) with forty micrograms of Clone 123 plasmid DNA, giving five payloads of 8 µg DNA, using a derivative of known methods for coating microparticles. Potter et al. (1984) *Proc. Natl. Acad. Sci. USA* 81:7161, Klein et al. (1987) *Nature* 327:70, and Williams et al. (1991) *Proc. Natl*. *Acad. Sci. USA* 88:2726.

More particularly, prior to coating, the tungsten particles were sterilized and brought into suspension. A 50 mg sample of 1.048 µm (median diameter) tungsten microprojectiles (M-17, GTE/Sylvania, Towanda, PA, USA) was weighed into a 1.5 cc Eppendorf tube and then sterilized in 100% ethanol (EtOH). In order to disperse the microprojectiles (disrupt particle aggregates), the sterilized solution was sonicated thoroughly by contacting the outside of the Eppendorf tube with the probe of a sonicator. The dispersed tungsten particles were centrifuged and the supernatant removed. The tungsten particles were resuspended in 1 cc sterile distilled water and centrifuged for two cycles, and then stored in 1 cc sterile distilled water until coating.

The plasmid DNA was absorbed to the tungsten microprojectiles by adding 20 µL of the DNA (1 mg/mL) to 40 µL of the suspension of tungsten particles prepared above. The suspension was vortexed to ensure adequate mixing of the reagents. The following reagents were then added, in the order given, with vortexing after each addition: 253 µL CaCl₂ (2.5 M); 50 µL spermidine (0.10 M, stored frozen); and 207 µL sterile distilled H₂O. The final mixture was vortexed for 10 minutes at 4 °C. The DNA-coated tungsten microprojectiles were then centrifuged at 500 G for 5 minutes. After centrifugation, all supernatant was carefully removed and 100 µL of 70% EtOH added. The coated particles were again centrifuged, all supernatant removed, and the final preparation resuspended in 30 µL of 100% EtOH.

The above method resulted in suitable quantities of DNA-coated tungsten particles to allow for 4 to 5 deliveries by the needleless syringe. The above-noted reagent quantities can, of course, be varied to provide different loadings of DNA in accordance with known methods. The volume and molar concentration (M) of the stock solutions used to coat tungsten with DNA are given below in Table 2.

**Table 2**

| Component | Quantity (µL) |
|---|---|
| Tungsten particles (50 mg/mL) | 40 |
| Clone 123 (2.7 µg/µL) | 14.8 |
| Ca Cl₂ (2.5 M) | 253 |
| Spermidine (0.1 M) | 50 |
| distilled H₂O | 212.2 |

For transformation, 6 cm diameter culture dishes were seeded with 5 x 10⁵ male human fibroblast HT1080 cells 24 hours before transfection. Two replicate dishes were prepared for each of the treatments, and two negative control plates were also prepared.

The microparticles and the tungsten-coated particles were then delivered to cells using a needleless syringe as described above. The syringe included a 4.5 mL reservoir chamber with plunger type valve, a helium gas reservoir, a Mach 2 nozzle and 12 µm Mylar sheet hand-punched into 6 mm diameter diaphragms.

In particular, mylar diaphragms were first sterilized by singly layering between pieces of filter paper, stacked one atop the other, wrapped in aluminum foil and sealed completely with autoclave tape to ensure that no water entered the filter paper/diaphragm stack during the autoclave process. This was placed inside a beaker covered with aluminum foil and placed in an autoclave chamber.

Two 12 µm Mylar diaphragms of 6 mm diameter were used in the membrane cassette. One milligram and one-half milligram payloads of the freeze-dried DNA powder were loaded onto the lower membrane in the cassette. This payload was then covered with the other pieces of the cassette and the remaining diaphragm. Only preparations 2 and 3 were used in the experiment because of the difficulty in weighing out small masses accurately. Another five of the cassettes were each loaded with 5 µL of the DNA/tungsten particle suspension. All the above quantities gave a mass of about 8 µg of DNA being delivered in each shot regardless of particle formulation used.

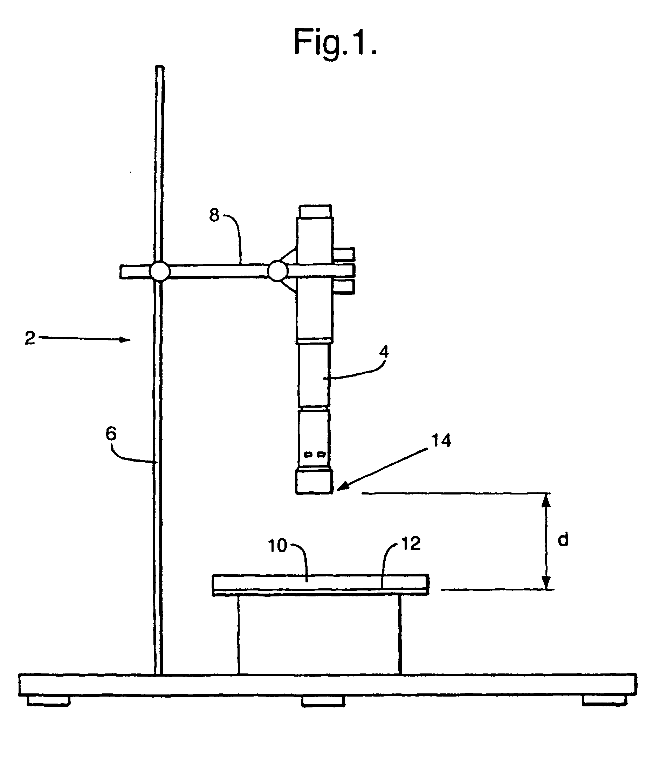

Referring now to Figure 1, a delivery apparatus 2 was assembled which contained a needleless syringe 4 loaded with a cassette as described above. The needleless syringe 4 was arranged on a ring stand 6 using a standard tube clamp 8 to hold the syringe in position relative to a culture dish 10 seeded with the HT1080 cells 12. The distance between the downstream terminus 14 of the needleless syringe 4 and the cells 14 in the culture dish 10 was measured to affix a target distance, generally indicated at d. In order to optimize the parameters for delivery of the freeze-dried DNA, either the target distance d was varied over a constant delivery pressure, or the delivery pressure was varied over a constant target distance. In particular, the DNA preparations were fired from a target distance ranging from 20 to 60 mm using helium driver gas pressures ranging from 30 to 50 bar.

After transformation, cells were incubated for 2 days, stained, and then transient assays with X-gal were performed to determine transformation efficiencies using previously described methods. Murray, E.J. (ed) (1991) *Methods in Molecular Biology: Gene Transfer and Expression Protocols,* Vol. 7, Humana Press, Clifton, New Jersey. Specifically, transformation efficiency was assessed by counting the number of blue-stained cells. The delivery parameters and transformation results are depicted below in Table 3. Blast effect was rated from 1 point for quite small (diameter of dead cell zone being approximately 5-8 mm) to five points for very large (diameter of cell zone being greater than 30 mm). As can be seen, transformation by the plasmid/trehalose powder preparation of the present invention was on the same order as that observed for the metallic particles.

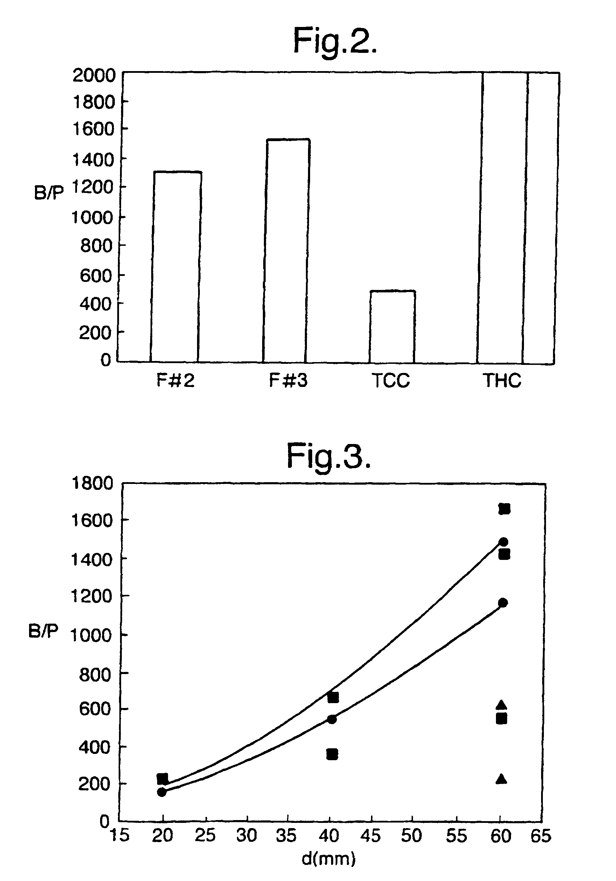

As shown in Figure 2, optimal transformation results were seen with the particulate plasmid/trehalose preparation when delivered using 30 bar pressure at a target distance of 60 mm. More particularly, Figure 2 provides a direct comparison of the transformation efficiency obtained by delivery of the particulate nucleic acid preparation (both preparations 2 and 3) with historical and contemporary deliveries of DNA-coated tungsten particles. Referring now to Figure 3, data obtained for deliveries at 30 bar are depicted in a graph which presents transformation efficiency as a function of target distance. As can be seen, the optimal target distance for the number 2 and 3 preparations (referred to as F#2 and F#3, respectively) was not reached; however, transformation efficiency did substantially increase with increased target distances. Further, when deliveries were carried out at the maximum distance tested (60 mm), transformation efficiencies obtained with the particulate DNA formulations (F#2 and F#3) were appreciably better than those observed with the DNA-coated tungsten controls.

In Figs. 2 and 3, the following abbreviations (that are not defined above) apply:
B/P Blue Cell Count per Plate
TCC Tungsten Contemporary Control
THC Tungsten Historical Control

Further, in Fig. 3, the points plotted are as follows:
- F#2
- F#3
- Tungsten (the point is obscured, at d = 20)

### Reference Example 2

The following studies were carried out to assess the ability to deliver a powdered nucleic acid composition to a test subject *in vivo* using the methods of the invention.

Plasmid Vector Construct: The pGREEN-1 vector construct, which contains the Green Fluorescent Protein (GFP) gene under the control of a CMV promoter, was used so that gene expression could be assessed directly by UV microscopy of histological sections from treated tissue samples.

Powdered Nucleic Acid Compositions: A powdered nucleic acid composition was prepared as follows. A mixture was formed by combining pGREEN-1 vector plasmid with trehalose sugar to obtain a 1µg:1mg (w/w) DNA-sugar composition. This composition was lyophilized, compressed, ground, and then sieved, using the techniques described hereinabove. The resulting condensed nucleic acid composition had an average particle size ranging from about 38-75 µm.

Administrations: C57BL/10 mice were treated with 1 mg of the particulate composition via needleless injection. The composition was delivered to a suitably prepared target skin surface, and histological sections were taken from the target site 24 hours after administration. GFP expression was determined directly using UV microscopy. As a result of the administrations, GFP expression was seen in the treated skin tissue, confirming successful *in vivo* delivery of the powdered nucleic acid composition to the target skin, and the subsequent transfection of host cells and expression of the GFP gene therefrom.

In another study, plasmids containing either a human Growth Hormone (hGH) or β-galactosidase (β-Gal) expression cassette were lyophilized with trehalose excipient to form nucleic acid formulations, which were compressed, ground, and then sieved, using the above-described techniques. The resulting condensed nucleic acid compositions had an average particle size ranging from about 38-75 µm.

Female pigs (weighing 20-25 kg) were anesthestised with halothane, and the belly skin was clipped to reveal a suitable target site. The above powdered nucleic acid compositions were individually administered to the prepared target site in 0.1 µg (hGH) or 1 µg (β-Gal) doses via a needleless injection device (delivery pressure of 60 bar). The target sites were biopsied 24 hours after treatment, and histological sections were analyzed for human growth hormone or β-Gal expression. Although no hGH expression was seen within the detection limits of the assay, a moderate degree of β-Gal expression was seen in the treated sites. The lack of detectable hGH expression in this study is due, presumably, to the low loading density of the nucleic acid (0.1 µg) in the composition.

**Table 3**

| Shot | Formulation | Target Distance | Pressure | Blue Cell Count | Blast effect |
|---|---|---|---|---|---|
| A1 | Tungsten | 60 | 30 | 224 | 2 |
| A2 | Tungsten | 60 | 30 | 596 | 2 |
| A3 | Tungsten | 60 | 30 | 575 | 2 |
| A4 | Tungsten | 60 | 30 | 581 | 2 |
| A5 | Tungsten | 60 | 30 | - | - |
| B1 | #3 trehalose | 20 | 30 | 155 | 3 |
| B2 | #3 trehalose | 20 | 30 | 227 | 3 |
| C1 | #3 trehalose | 40 | 30 | 654 | 2 |
| C2 | #3 trehalose | 40 | 30 | 643 | 2 |
| D1 | #3 trehalose | 40 | 50 | 394 | 4 |
| D2 | #3 trehalose | 40 | 50 | 175 | 5 |
| E1 | #3 trehalose | 60 | 30 | 1416 | 1 |
| E2 | #3 trehalose | 60 | 30 | 1654 | 1 |
| F1 | #3 trehalose | 60 | 50 | 408 | 3 |
| F2 | #3 trehalose | 60 | 50 | 486 | 3 |
| G1 | #2 trehalose | 20 | 30 | 166 | 3 |
| G2 | #2 trehalose | 20 | 30 | 180 | 3 |
| H1 | #2 trehalose | 20 | 50 | 129 | 4 |
| H2 | #2 trehalose | 20 | 50 | 53 | 4 |
| J1 | #2 trehalose | 40 | 30 | 347 | 2 |
| J2 | #2 trehalose | 40 | 30 | 546 | 2 |
| K1 | #2 trehalose | 40 | 50 | 377 | 4 |
| K2 | #2 trehalose | 40 | 50 | 198 | 4 |
| L1 | #2 trehalose | 60 | 30 | 1451 | 1 |
| L2 | #2 trehalose | 60 | 30 | 1164 | 1 |
| M1 | #2 trehalose | 60 | 50 | 409 | 3 |
| M2 | #2 trehalose | 60 | 50 | 336 | 3 |

## Claims

1. Use of a nucleic acid molecule and a carbohydrate as sole carrier for the manufacture of a medicament in the form of particles for use in therapy by needleless administration to skin or mucosal tissue, wherein
- the nucleic acid molecule comprises a nucleotide sequence encoding an immunogen,
- control sequences capable of effecting the expression of said nucleotide sequence are operably linked thereto, and
- said particles have a size ranging from 10 to 150 µm and a density ranging from 0.8 to 3.0 g/cm³.

2. Use according to claim 1, wherein said nucleic acid molecule is a DNA sequence.

3. Use according to claim 1 or 2, wherein said nucleic acid molecule is a plasmid.

4. Use according to any one of the preceding claims, wherein said carbohydrate is trehalose.

5. Use according to any one of the preceding claims, wherein said particles have a size ranging from 20 to 60 µm.

6. Use according to any one of the preceding claims, wherein said particles have a density ranging from 0.9 to 1.5 g/cm³.

7. Use according to any one of the preceding claims, wherein said nucleotide sequence encodes an antigen capable of eliciting a humoral immune response.

8. Use according to any one of the preceding claims, wherein said nucleotide sequence encodes an antigen capable of eliciting a cellular immune response.

9. Use according to any one of the preceding claims, wherein said particles are administered at a momentum density of between 2 and 10 kg/sec/m.

10. A needleless syringe comprising, as the active component to be delivered, particles as defined in any one of claims 1 to 8.

## Patentansprüche

1. Verwendung eines Nukleinsäure-Moleküls und eines Kohlenhydrats als alleinigem Träger für die Herstellung eines Medikaments in Form von Partikeln zur Anwendung in der Therapie durch nadellose Verabreichung an Haut- oder Schleimhautgewebe, wobei
- das Nukleinsäure-Molekül eine Nukleotidsequenz umfasst, die für ein Immunogen codiert,
- Kontrollsequenzen, die zum Bewirken der Expression der Nukleotidsequenz fähig sind, funktionsfähig daran geknüpft sind, und
- diese Partikel eine Größe im Bereich von 10 bis 150 µm und eine Dichte im Bereich von 0,8 bis 3,0 g/cm³ aufweisen.

2. Verwendung nach Anspruch 1, wobei das Nukleinsäure-Molekül eine DNA-Sequenz ist.

3. Verwendung nach Anspruch 1 oder 2, wobei das Nukleinsäure-Molekül ein Plasmid ist.

4. Verwendung nach einem der vorangehenden Ansprüche, wobei das Kohlenhydrat Trehalose ist.

5. Verwendung nach einem der vorangehenden Ansprüche, wobei die Partikel eine Größe im Bereich von 20 bis 60 µm aufweisen.

6. Verwendung nach einem der vorangehenden Ansprüche, wobei die Partiel eine Dichte im Bereich von 0,9 bis 1,5 g/cm³ aufweisen.

7. Verwendung nach einem der vorangehenden Ansprüche, wobei die Nukleotidsequenz für ein Antigen codiert, das zum Hervorrufen einer humoralen Immunantwort fähig ist.

8. Verwendung nach einem der vorangehenden Ansprüche, wobei die Nukleotidsequenz für ein Antigen codiert, das zum Hervorrufen einer zellulären Immunantwort fähig ist.

9. Verwendung nach einem der vorangehenden Ansprüche, wobei die Partikel bei einer Irnpulsdichte von zwischen 2 und 10 kg/sec/m verabreicht werden.

10. Nadellose Spritze, umfassend, als dem zu verabreichenden Wirkstoff, Partikel, wie in einem der Ansprüche 1 bis 8 definiert.

## Revendications

1. Utilisation d'une molécule d'acide nucléique et d'un glucide en tant que support unique pour la fabrication d'un médicament sous la forme de particules pour l'utilisation en thérapie par administration sans aiguille à la peau ou un tissu muqueux, dans laquelle
- la molécule d'acide nucléique comprend une séquence nucléotidique codant un immunogène,
- des séquences régulatrices capables d'effectuer l'expression de ladite séquence nucléotidique sont liées de façon opératoire à celle-ci, et
- lesdites particules ont une taille allant de 10 à 150 µm et une masse volumique allant de 0,8 à 3,0 g/cm³.

2. Utilisation selon la revendication 1, dans laquelle ladite molécule d'acide nucléique est une séquence d'ADN.

3. Utilisation selon la revendication 1 ou 2, dans laquelle ladite molécule d'acide nucléique est un plasmide.

4. Utilisation selon l'une quelconque des revendications précédentes, dans laquelle ledit glucide est le tréhalose.

5. Utilisation selon l'une quelconque des revendications précédentes, dans laquelle lesdites particules ont une taille allant de 20 à 60 µm.

6. Utilisation selon l'une quelconque des revendications précédentes, dans laquelle lesdites particules ont une masse volumique de 0,9 à 1,5 g/cm³.

7. Utilisation selon l'une quelconque des revendications précédentes, dans laquelle ladite séquence nucléotidique code un antigène capable de susciter une réponse immunitaire humorale.

8. Utilisation selon l'une quelconque des revendications précédentes, dans laquelle ladite séquence nucléotidique code un antigène capable de susciter une réponse immunitaire cellulaire.

9. Utilisation selon l'une quelconque des revendications précédentes, dans laquelle lesdites particules sont administrées à une densité cinétique (momentum density) entre 2 et 10 kg/s/m.

10. Seringue sans aiguille comprenant, en tant que constituant actif devant être délivré, des particules telles que définies dans l'une quelconque des revendications 1 à 8.
